# EUROPEAN PATENT SPECIFICATION

(11) **EP 1 631 244 B1**
(45) Date of publication and mention of the grant of the patent: **27.02.2008**
(21) Application number: 04754557.9
(22) Date of filing: 07.06.2004
(51) Int. Cl.: A61K 8/18, A61K 8/99

(54) **HAIR OR SKIN CONDITIONING COMPOSITION COMPRISING HYDROPHOBICALLY MODIFIED CATIONIC THICKENING POLYMER**
HAAR- ODER HAUT-KONDITIONIERUNGSZUSAMMENSETZUNG MIT HYDROPHOBISCH MODIFIZIERTEM KATIONISCH VERDICKENDEM POLYMER
COMPOSITION REVITALISANTE POUR LES CHEVEUX OU LA PEAU A BASE DE POLYMERE EPAISSISSANT CATIONIQUE HYDROPHOBIQUEMENT MODIFIE

(30) Priority: 06.06.2003 US 476707 P
(43) Date of publication of application: 08.03.2006
(73) Proprietor: THE PROCTER & GAMBLE COMPANY, Cincinnati, Ohio 45202 (US)
(72) Inventor: ASARI, Satomi, Kobe, Hyogo 658-0054 (JP); GUSKEY, Susan, Marie, Loveland, OH 45140 (US); KHAN, Golam, Faruque, Darien, CT 06820 (US); JOHNSON, Eric, Scott, Hamilton, OH 45011 (US); WELLS, Robert, Lee, Cincinnati, OH 45229 (US)
(74) Representative: Kohol, Sonia
(86) International application number: PCT/US2004/017986
(87) International publication number: WO 2004/108099

(56) References cited:
- EP-A- 0 412 705
- WO-A-01/34103
- WO-A-01/76543
- WO-A-99/02122
- WO-A-20/04032887

## Description

### FIELD OF THE INVENTION

The present invention relates to a conditioning composition comprising a hydrophobically modified cationic thickening polymer, a cationic or nonionic surfactant, and a hydrophobically modified silicone. The conditioning composition of the present invention has a suitable rheology for conditioning compositions and provides conditioning benefits. The composition is especially suitable for hair care products such as hair conditioning products for rinse-off use.

### BACKGROUND OF THE INVENTION

A variety of conditioning compositions such as hair conditioning compositions and skin conditioning compositions have been used for a variety of substances such as hair and skin. A common method of providing conditioning benefits is through the use of conditioning agents such as cationic surfactants and polymers, high melting point fatty compounds, low melting point oils, silicone compounds, and mixtures thereof. Most of these conditioning agents are known to provide various conditioning benefits. For example, some cationic surfactants, when used together with some high melting point fatty compounds, are believed to provide a gel matrix which has a suitable rheology for conditioning compositions and which is suitable for providing a variety of conditioning benefits, especially when used for hair care products, such as slippery feel, softness and reduced tangling on wet hair and softness and moisturized feel on the hair when they are dried.

There exists a need for achieving the suitable rheology for conditioning compositions by other methods than forming the above gel matrix, while maintaining the conditioning benefits of the gel matrix.

Additionally, most of the above conditioning agents are also known to make the composition opaque. Thus, there is a need for conditioning compositions having a clear product appearance i.e., transparent or translucent product appearance.

Furthermore, most of the above conditioning agents are also known to weigh down the hair, when these conditioning agents are included in hair care compositions. For consumers who desire maintaining or increasing hair volume such as consumers having fine hair, the hair weighing down is not desirable. Thus, there is a need for hair conditioning compositions which do not weigh down the hair while providing conditioning benefits.

There also exists a need for conditioning compositions which consumers feel are easy to rinse-off while providing conditioning benefits, when the compositions are used in a form of rinse-off products. Conditioner compositions containing the above gel matrix also provide long-lasting slippery feel when rinsing the substance. Thus, there is a need for conditioning compositions which can easily leave the substance with a clean feel when rinsing the substance, while depositing sufficient amount of conditioning agents on the substance.

Based on the foregoing, there remains a need for conditioning compositions which have a suitable rheology for conditioning compositions by other methods than a gel matrix comprised by cationic surfactants and high melting point fatty compounds, while providing conditioning benefits, especially softness and reduced tangling on wet hair when used for hair care products such as hair conditioning products. There is also a need for such conditioning compositions which are suitable for providing further benefits such as, clear product appearance, not weighing down the hair, and easy to rinse-off feel, while providing the above rheological and conditioning benefits.

None of the existing art provides all of the advantages and benefits of the present invention.

### SUMMARY OF THE INVENTION

The present invention is directed to a hair or skin composition comprising by weight:
(a) from 0.01% to 5% of a Hydrophobically modified cationic thickening polymer; wherein the hydrophobically modified cationic thickening polymer comprises a hydrophilic polymer backbone and a hydrophobic substitution group; wherein the hydrophilic polymer backbone is selected from the group consisting of a hydrophilic cellulose, a hydrophilic guar gum, a hydrophilic synthetic polymer, and mixtures thereof; and wherein the hydrophobic substitution group is cationic and has the following formula (ii): 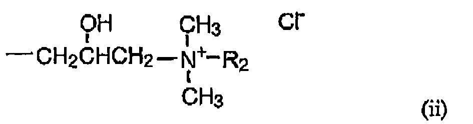 wherein R₂ is an alkyl group having from 8 to 22 carbons, and the hydrophobically modified cationic thickening polymer further comprises a cationic substitution group having the following formula (i): 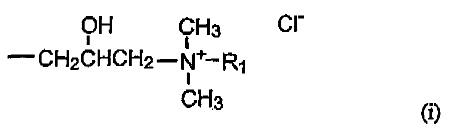 wherein R₁ is an alkyl having from 1 to 7 carbons.
(b) from 0.05% to 10% of a surfactant selected from the group consisting of cationic surfactant, nonionic surfactant, and mixtures thereof;
(c) from 0.1% to 10% of a hydrophobically modified silicone; and
(d) an aqueous carrier.

These and other features, aspects, and advantages of the present invention will become better understood from a reading of the following description, and appended claims.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentage, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

### COMPOSITIONS

The hair or skin composition of the present invention comprises by weight:
(a) from 0.01% to 5% of a hydrophobically modified cationic thickening polymer; wherein the hydrophobically modified cationic thickening polymer comprises a hydrophilic polymer backbone and a hydrophobic substitution group; wherein the hydrophilic polymer backbone is selected from the group consisting of a hydrophilic cellulose, a hydrophilic guar gum, a hydrophilic synthetic polymer, and mixtures thereof; and wherein the hydrophobic substitution group is cationic and has the following formula (ii): 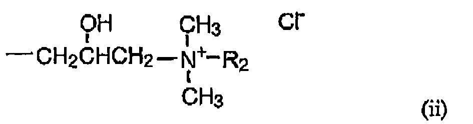 wherein R₂ is an alkyl group having from 8 to 22 carbons, and the hydrophobically modified cationic thickening polymer further comprises a cationic substitution group having the following formula (i): 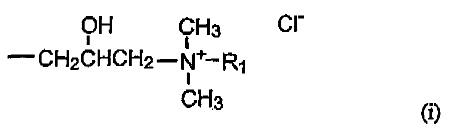 wherein R₁ is an alkyl having from 1 to 7 carbons.
(b) from 0.05% to 10% of a surfactant selected from the group consisting of cationic surfactant, nonionic surfactant, and mixtures thereof;
(c) from 0.1% to 10% of a hydrophobically modified silicone; and
(d) an aqueous carrier.

The conditioning compositions of the present invention have a suitable rheology for conditioning compositions and provide conditioning benefits, especially softness and reduced tangling on wet hair when used for hair care products such as hair conditioning products. The conditioning compositions of the present invention are suitable for providing further benefits such as clear product appearance, not weighing down the hair, and easy to rinse-off feel, while providing the above rheological and conditioning benefits. Thus, the composition of the present invention can provide clear product appearance in addition to the above rheological and conditioning benefits. When used for hair care products, the composition of the present invention provides the above rheological and conditioning benefits while not weighing down the hair. Furthermore, when used in a form of rinse-off products, the composition of the present invention can provide easy to rinse-off feel while providing the above rheological and conditioning benefits.

Without intending to be limited by theory, it is believed that the hydrophobically modified cationic thickening polymers interact with the surfactant and the hydrophobically modified silicone to form a physically crosslinked matrix which can provide a suitable rheology for conditioning compositions without the existence of a gel matrix comprised by cationic surfactants and high melting point fatty compounds, while providing conditioning benefits, especially softness and reduced tangling on wet hair when used for hair care products such as hair conditioning products.

Preferably, the composition of the present invention is substantially free of water-insoluble oily compound selected from hydrocarbons, fatty compounds, and mixtures thereof. In the present invention, the composition being "substantially free of water-insoluble oily compound" means that the composition includes 1.0% or less, preferably 0.5% or less, more preferably 0.1% or less, still more preferably 0% of water-insoluble oily compounds. The water-insoluble oily compounds herein are those having a solubility in water at 25°C of less than 1g/100g water, preferably less than 0.5g/100g water, more preferably less than 0.1g/100g water. Such water-insoluble oily compounds arc selected from hydrocarbons, fatty compounds, and mixtures thereof. Such hydrocarbons include, for example, poly α-olefin oils, paraffins, waxes, and mixtures thereof. Such fatty compounds include, for example, fatty alcohols such as cetyl alcohol and stearyl alcohol, fatty acids such as stearic acid, fatty alcohol derivatives and fatty acid derivatives such as esters and ethers thereof, and mixtures thereof.

Preferably the composition of the present invention is transparent or translucent, and more preferably transparent. In the present invention, the composition being "transparent" means that the composition has a transmittance of 50% or more, preferably 65% or more, more preferably 80% or more. In the present invention, the composition being "translucent" means that the composition has a transmittance of from 25% to 50%, preferably from 35% to 50%. The transmittances are measured at 600nm using UV-1601 which is a UV-visible spectrophotometer available from Shimadzu.

Preferably, the composition is substantially free of anionic compounds. Anionic compounds herein include anionic surfactants and anionic polymers. In the present invention, the composition being "substantially free of anionic compounds" means that the composition includes 1% or less, preferably 0.5% or less, more preferably 0% of anionic compounds.

### HYDROPHOBICALLY MODIFIED CATIONIC THICKENING POLYMER

The conditioning composition of the present invention comprises a hydrophobically modified cationic thickening polymer. The thickening polymers useful herein are those which can provide appropriate viscosity and rheology properties to the composition, so that the composition of the present composition has a suitable viscosity, preferably from 100 mPa.s⁻¹(cps) to 100,000 mPa.s⁻¹(cps), more preferably from 1,000 mPa.s⁻¹(cps) to 50,000 mPa.s⁻¹(cps), still more preferably from 2,000 mPa.s⁻¹(cps) to 50,000 mPa.s⁻¹(cps), even more preferably from 5,000 mPa.s⁻¹(cps) to 20,000 mPa.s⁻¹(cps). The viscosity herein can be suitably measured by Brookfield RVT at a shear rate or 2·s⁻¹ at 26.7°C.

Preferably, the hydrophobically modified cationic thickening polymers useful herein is hydrophilic, and has a solubility in water at 25°C of at least 0.25g/100g water, more preferably at least 2g /100g water, still more preferably at least 5g/100g water, even more preferably at least 15g/100g water.

The thickening polymers are included in the composition of the present invention at a level by weight of from 0.01% to 5.0%, preferably from 0.1% to 3.0%, more preferably from 0.3% to 2.0%, still more preferably from 0.4% to 0.8%.

The hydrophobically modified cationic thickening polymer useful herein comprises a hydrophilic polymer backbone and a hydrophobic substitution group. The hydrophobically modified cationic thickening polymer useful herein also comprises a cationic substitution group.

The hydrophilic polymer backbone useful herein include a hydrophilic cellulose, a hydrophilic guar gum, a hydrophilic synthetic polymer, and mixtures thereof. The hydrophilic cellulose useful herein include, for example, hydroxymethyl cellulose, hydroxyethyl cellulose, hydroxyethyl ethylcellulose, hydroxypropyl cellulose, hydroxypropyl methylcellulose, hydroxybutyl cellulose, and mixtures thereof. The hydrophilic synthetic polymer useful herein include, for example, those containing hydrophilic monomers. Such monomers useful herein include, for example, those having polyalkylene oxide groups containing from 1 to 50 alkylene oxides, preferably from 5 to 45, more preferably from 10 to 40. The alkylene oxides are preferably selected from ethylene oxides, propylene oxides, and mixtures thereof. Preferred are those selected from the group consisting of vinyl alkoxylates, PEG 10-40 (meth)acrylates, and mixtures thereof. Among the above hydrophilic polymer backbones, preferred is hydroxyethyl cellulose.

The hydrophobically modified cationic thickening polymer further comprises a cationic substitution group of the formula (i): 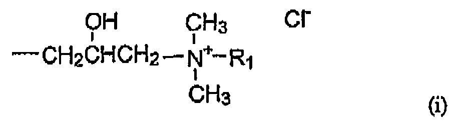 wherein R₁ is an alkyl having from 1 to 7 carbon atoms, preferably from 1 to 3 carbon atoms, more preferably R₁ is methyl.

The hydrophilic polymer backbone is further substituted with a hydrophobic substitution group of the formula (ii): 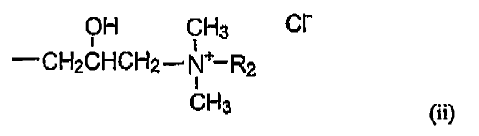 wherein R₂ is an alkyl group having from 8 to 22 carbon atoms, preferably from 10 to 18 carbon atoms, more preferably R₂ is an alkyl group having 12 carbon atoms.

The hydrophobically modified cationic thickening polymers useful herein have a molecular weight of preferably from 50,000 to 10,000,000, more preferably from 75,000 to 2,000,000, still more preferably from 100,000 to 500,000. The hydrophobically modified cationic thickening polymers useful herein have a cationic charge density of preferably from 0.1 meq/g to 2.0 meq/g, more preferably from 0.3 meq/g to 1.5 meq/g, still more preferably from 0.5 to 1.2 meq/g. The cationic charge can result from both of the cationic substitution group having the formula (i) and the hydrophobic substitution group having the formula (ii). The hydrophobically modified cationic thickening polymers useful herein also have a level of hydrophobic substitution of preferably from 0.05 to 0.15, more preferably from 0.07 to 0.15, still more preferably from 0.10 to 0.12. By "a level of hydrophobic substitution", what is meant is mols of hydrophobic substitutions (corresponding to R₂ group in the formula (ii) above) per one glucose, one mannose, or a hydrophilic systhetic monomer unit. Especially, when the hydrophobically modified cationic thickening polymers have a molecular weight of less than 500,000, they have a level of hydrophobic substitution of preferably from 0.10 to 0.15, more preferably from 0.10 to 0.12.

The hydrophobically modified cationic thickening polymer of the present invention has both the cationic substitution group having the formula (i) and the hydrophobic substitution group having the formula (ii). By these substitution groups, the hydrophobically modified cationic thickening polymers of the present invention have a higher cationic charge density and a higher hydrophobic substitution level. It is believed that; among thickening polymers of the above formula, those having a higher molecular weight provide more viscous composition, those having a higher cationic charge density provide less viscous composition, and those having a higher hydrophobic substitution provide more viscous composition. It is also believed that; by the selection of molecular weight, cationic charge density, and hydrophobic substitution level, the thickening polymer of the present invention can provide balanced viscosity and rheology suitable for conditioning compositions.

The composition of the present invention can contain a mixture of hydrophobically modified cationic thickening polymers. Preferably, the mixture contains a first hydrophobically modified cationic thickening polymer having a molecular weight of from 50,000 to 500,000, and a second hydrophobically modified cationic thickening polymer having a molecular weight of from 500,000 to 10,000,000. Such mixture can provide a balanced benefit in view of rheology and conditioning properties, at a lower total level of thickening polymers.

A preferred hydrophobically modified cationic thickening polymer is a hydrophobically modified cationic hydroxyethyl cellulose polymer having the following formula: 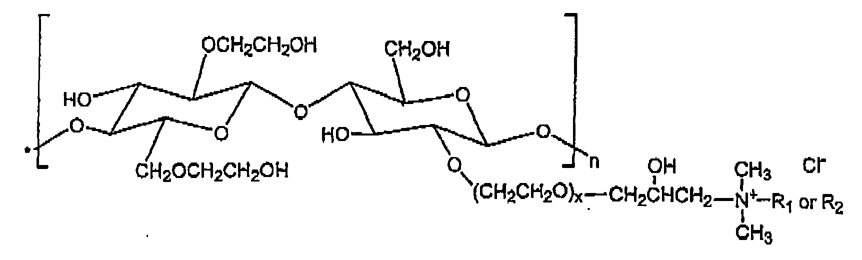 wherein R₁ and R₂ are defined above; n is an integer of from 200 to 7,000, preferably from 250 to 5,000; x is 0 or an integer of from 1 to 6, preferably from 1 to 3. Especially preferred hydrophobically modified cationic thickening polymers useful herein include, for example, a hydrophobically modified cationic cellulose available from Amerchol.

### SURFACTANT

The compositions of the present invention comprise a surfactant. The surfactant is included in the composition at a level by weight of from 0.05% to 5%, preferably from 0.1% to 3%, more preferably from 0.2% to 2%, still more preferably from 0.3 to 1%.

Preferably, in view of the desire for a transparent or translucent appearance, the surfactant system is substantially soluble in the composition at the level used. By "substantially soluble" surfactant system, what is meant is that the composition has a transmittance of 50% or more, preferably 65% or more, more preferably 80% or more at 25°C when containing the surfactant system at the level used.

The surfactant useful herein is selected from the group of consisting of a cationic surfactant, a nonionic surfactant, and mixtures thereof.

A variety of cationic surfactants which can be used in the composition of the present invention arc described below. Among them, preferred are mono-long alkyl trimethyl ammonium salts. The mono-long alkyl trimethyl ammonium salts useful herein are those in which the alkyl has from 12 to 28 carbon atoms, preferably from 16 to 22 carbon atoms. Highly preferred mono-long alkyl trimethyl ammonium salts arc, for examples, cetyl trimethyl ammonium chloride, stearyl trimethyl ammonium chloride.

Cationic surfactants useful herein include, for example, those corresponding to the general formula (I): 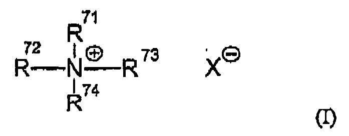 wherein at least one of R⁷¹, R⁷², R⁷³ and R⁷⁴ is selected from an aliphatic group of from 8 to 30 carbon atoms or an aromatic, alkoxy, polyoxyalkylene, alkylamido, hydroxyalkyl, aryl or alkylaryl group having up to about 22 carbon atoms, the remainder of R⁷¹, R⁷², R⁷³ and R⁷⁴ are independently selected from an aliphatic group of from 1 to 22 carbon atoms or an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The aliphatic groups can contain, in addition to carbon and hydrogen atoms, ether linkages, and other groups such as amino groups. The longer chain aliphatic groups, e.g., those of 12 carbons, or higher, can be saturated or unsaturated. Preferred is when R⁷¹, R⁷², R⁷³ and R⁷⁴ arc independently selected from C₁ to C₂₂ alkyl. Nonlimiting examples of cationic surfactants useful in the present invention include the materials having the following CTFA designations: quaternium-8, quaternium-14, quaternium-18, quaternium-18 methosulfate, quaternium-24, and mixtures thereof.

Among the cationic surfactants of general formula (I), preferred are those containing in the molecule at least one alkyl chain having at least 16 carbons. Nonlimiting examples of such preferred cationic surfactants include: behenyl trimethyl ammonium chloride available, for example, with tradename Genamine KDMP from Clariant, with tradename INCROQUAT TMC-80 from Croda, and with tradename ECONOL TM22 from Sanyo Kasei; cetyl trimethyl ammonium chloride available, for example, with tradename CTAC 30KC from KCI, and with tradename CA-2350 from Nikko Chemicals; stearyl trimethyl ammonium chloride available, for example, with tradename Genamine STACP from Clariant; olealkonium chloride available, for example, with tradename Incroquat O-50 from Croda; hydrogenated tallow alkyl trimethyl ammonium chloride, dialkyl (14-18) dimethyl ammonium chloride, ditallow alkyl dimethyl ammonium chloride, dihydrogenated tallow alkyl dimethyl ammonium chloride, distearyl dimethyl ammonium chloride, dicctyl dimethyl ammonium chloride, di(behenyl/arachidyl) dimethyl ammonium chloride, dibehenyl dimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, and N-(stearoyl colamino formyl methyl) pyridinium chloride.

Also preferred arc hydrophilically substituted cationic surfactants in which at least one of the substituents contain one or more aromatic, ether, ester, amido, or amino moieties present as substituents or as linkages in the radical chain, wherein at least one of the R71-R74 radicals contain one or more hydrophilic moieties selected from alkoxy (preferably C1-C3 alkoxy), polyoxyalkylene (preferably C1-C3 polyoxyalkylene), alkylamido, hydroxyalkyl, alkylester, and combinations thereof. Preferably, the hydrophilically substituted cationic conditioning surfactant contains from 2 to 10 nonionic hydrophile moieties located within the above stated ranges. Highly preferred hydrophilically substituted cationic surfactants include dialkylamido ethyl hydroxyethylmonium salt, dialkylamidoethyl dimonium salt, dialkyloyl ethyl hydroxyethylmonium salt, dialkyloyl ethyldimonium salt, and mixtures thereof; for example, commercially available under the following tradenames; VARISOFT 110, VARISOFT 222, VARIQUAT K1215 and VARIQUAT 638 from Witco Chemical, MACKPRO KLP, MACKPRO WLW, MACKPRO MLP, MACKPRO NSP, MACKPRO NLW, MACKPRO WWP, MACKPRO NLP, MACKPRO SLP from McIntyre, ETHOQUAD 18/25, ETHOQUAD O/12PG, ETHOQUAD C/25, ETHOQUAD S/25, and ETHODUOQUAD from Akzo, DEHYQUAT SP from Henkel, and ATLAS G265 from ICI Americas. Babassuamidopropalkonium Chloride available from Croda under the tradename Incroquat BA-85 is also preferably used in the composition.

Amines are suitable as cationic surfactants. Primary, secondary, and tertiary fatty amines are useful. Particularly useful are tertiary amido amines having an alkyl group of from 12 to 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behanamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidylbehenylamine. Useful amines in the present invention are disclosed in U.S. Patent 4,275,055, Nachtigal, et al. These amines can also be used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid. The amines herein are preferably partially neutralized with any of the acids at a molar ratio of the amine to the acid of from 1 : 0.3 to 1 : 2, more preferably from 1 : 0.4 to 1 : 1.

A variety of nonionic surfactants can be used in the composition of the present invention. Such nonionic surfactants include, for example, polyoxyethylene alkyl ethers; polysorbates such as polysorbate-20 through 80; polyethylene glycol derivatives of glycerides such as polyethylene glycol derivatives of hydrogenated castor oil including PEG-40 through 100 hydrogenated castor oil, and polyethylene glycol derivatives of stearic acid including PEG-10 through 55 stearate. Among a variety of nonionic surfactants, preferred are polyoxyethylene alkyl ethers. Especially preferred polyoxyethylene alkyl ethers useful herein are those having a C12-C22 alkyl chain, and having the above HLB value. Highly preferred polyoxyethylene alkyl ethers include, for example, laureth series of compounds such as laureth-7 through 12; ceteth series of compounds such as ceteth-7 through 20; and pareth series of compounds such as pareth-9 through 15.

### HYDROPHOBICALLY MODIFIED SILICONE

The compositions of the present invention comprise a hydrophobically modified silicone. The hydrophobically modified silicone copolyol is included in the composition at levels by weight of from 0.1% to 10%, more preferably fiom 0.5% to 8%, still more preferably from 1% to 6%, even more preferably from 2% to 5%.

Among a variety of silicone compounds, preferred are those selected from the group consisting of (i) silicone nanoemulsion having an average particle size of 300nm or less, preferably 200um or less, more preferably 100nm or less when contained in the composition, (ii) silicone compound being substantially soluble in the composition, and (iii) mixtures thereof, in view of the desire for a transparent or translucent appearance. By "substantially soluble" silicone compound, what is meant is that the composition has a transmittance of 50% or more, preferably 65% or more, more preferably 80% or more at 25°C when containing the silicone compound at the level used.

Commercially available silicone nanoemulsion useful herein includes, for example, that with a tradename Silicone DC-8177 available from Dow Corning; quaternized silicone nanoemulsion with a tradename DC5-7133 available from Dow Corning; and amodimethicone nanoemulsion with a tradename XS65-B6413 available from General Electric.

With respect to substantially soluble silicone compounds, for example, following materials can be substantially soluble depending on the level of hydrophilic groups in their structure: silicone copolyols such as dimethicone copolyol with a tradename Silicone DC-5330 from Dow Corning; amino silicone copolyols such as those having an INCI name Bis (C13-15 Alkoxy) PG Amodimethicone available with a tradename DC2-8500 from Dow Corning; hydrophobically modified amino silicone copolyols; hydrophobically modified amido silicone copolyols; and quaternized silicones. Among these substantially soluble silicone compounds, more preferred are those selected from the group consisting of amino silicone copolyols, hydrophobically modified amino silicone copolyols, and hydrophobically modified amido silicone copolyols, and mixtures thereof, still more preferred are hydrophobically modified amido silicone copolyols in view of providing improved silicone deposition thus providing improved conditioning performance while meeting the desire for transparent or translucent appearance. Such hydrophobically modified amidomethicone copolyols have the following formula: 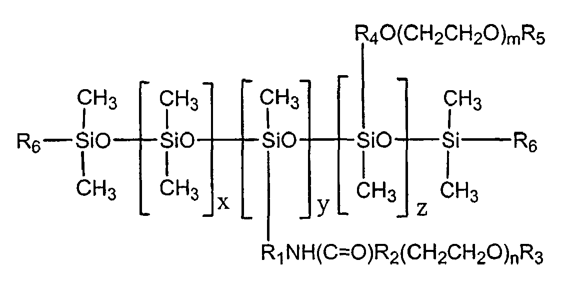 wherein R₁, R₂, R₄ are respectively C1-C3 alkyl, preferably ethyl; R₃ is an alkyl group having 8-22 carbon atoms, preferably 10-20 carbon atoms, more preferably 12-16 carbon atoms, even more preferably 12 carbon atoms; R₅ is H or C1-C3 alkyl, preferably methyl; R₆ is OH or CH₃, preferably methyl; n is an integer of 1-10, highly preferably 5; m is an integer of 2-20, highly preferably 12; n+m = 3-30, preferably 5-25, more preferably 8-20, even more preferably 17; x is an integer from 200 to 500, preferably from 300 to 400; y is an integer from 5 to 40, preferably from 10 to 30; and z is 0 or an integer from 1 to 30, preferably from 5 to 20. Commercially available hydrophobically modified amido silicone copolyols are, for example, those having an INCI name PEG-12 Methyl Ether/Lauroxy PEG-5 Amidopropyl Dimethicone available with a tradename Silicone BY16-906 from Dow Coming.

In view of providing improved conditioning benefits, it is preferred for the composition of the present invention to provide improved silicone deposition, even after rinsing-off the composition from the hair. For example, it is preferred for the compositions to provide silicone deposition of about 50ppm or more, more preferably 100ppm or more, still more preferably 200ppm or more, even more preferably 400ppm or more after rinsing-off the hair. The amount of the silicone deposition can be measured by a method consisting of: (i) a preparation of hair switch; and (ii) silicone deposition measurement

### (i) Preparation of hair switch

For the silicone deposition measurement, 2 gram hair switches are used. The hair switches are prepared by following steps:
(a) Providing five cycles of shampoo/conditioning treatments to the hair switch, each cycle of shampoo/conditioning treatment consisting of following steps:
   (a-1) Applying a shampoo at a level of 0.2cc and lathering the hair switch; and rinsing the hair switch;
   (a-2) Applying a shampoo again at a level of 0.2cc and lathering the hair switch; and rinsing the hair switch; and
   (a-3) Then providing conditioner treatment to the hair switch, the conditioner treatment consisting of applying a conditioner at a level of 0.2cc and treating the hair switch; and rinsing the hair switch; and
   (b) Then drying the hair switch.
   The hair switch is ready for the measurement of its silicone deposition amount.

### (ii) Silicone deposition measurement

The deposited silicone on the hair switch is extracted in an appropriate solvent. The extracts are then introduced into an atomic absorption/emission detector instrument and measured at the appropriate wavelength. The absorbance/emission value returned by the instrument is then converted to actual concentration (ppm) of silicone compound deposited on the hair through an external calibration curve obtained with known weights of a well characterized standard of the silicone compound under study.

### AQUEOUS CARRIER

The compositions of the present invention comprise an aqueous carrier. The level and species of the carrier are selected according to the compatibility with other components, and other desired characteristic of the product.

Carriers useful in the present invention include water and water solutions of lower alkyl alcohols. Lower alkyl alcohols useful herein are monohydric alcohols having 1 to 6 carbons, more preferably ethanol and isopropanol.

Preferably, the aqueous carrier is substantially water. Deionized water is preferably used. Water from natural sources including mineral cations can also be used, depending on the desired characteristic of the product. Generally, the compositions of the present invention comprise from 20% to 99%, preferably from 40% to 98%, and more preferably from 50% to 98% water.

The pH of the present composition is preferably from 3 to 9, more preferably from 3 to 7, still more preferably from 4 to 6. Buffers and other pH adjusting agents can be included to achieve the desirable pH.

### ADDITIONAL COMPONENTS

The composition of the present invention may include additional components, which may be selected by the artisan according to the desired characteristics of the final product and which are suitable for rendering the composition more cosmetically or aesthetically acceptable or to provide them with additional usage benefits.

### Humectant and/or Co-solvent

The compositions of the present invention may contain a humectant and/or co-solvent to help the surfactant system and/or silicone compound to be substantially soluble in the composition. The humectants and/or co-solvents herein are selected from the group consisting of polyhydric alcohols, water soluble alkoxylated nonionic polymers, and mixtures thereof. The humectants and/or co-solvents herein are preferably used at levels by weight of the composition of from 0.1% to 20%, more preferably from 0.5% to 5%.

Polyhydric alcohols useful herein include glycerin, sorbitol, propylene glycol, butylene glycol, hexylene glycol, ethoxylated glucose, 1, 2-hexane diol, hexanetriol, dipropylene glycol, erythritol, trehalose, diglycerin, xylitol, maltitol, maltose, glucose, fructose, sodium chondroitin sultate, sodium hyaluronate, sodium adenosin phosphate, sodium lactate, pyrrolidone carbonate, glucosamine, cyclodextrin, and mixtures thereof. Among them, preferred for the co-solvents are 1,2-hexane diol, hexylene glycol, butylene glycol, glycerine, and mixtures thereof.

Water soluble alkoxylated nonionic polymers useful herein include polyethylene glycols and polypropylene glycols having a molecular weight of up to 10,000 such as those with CTFA names PEG-4, PEG-8, PEG-12, PEG-20, PEG-150 and mixtures thereof.

### Additional silicone components

The composition of the present invention may contain additional silicone components. Such additional silicone components include, for example, polyalkyl siloxanes such as polydimethylsiloxane from General Electric Company in their TSF 451 series and from Dow Coming in their Dow Corning SH200 series; polyaryl siloxanes; polyalkylaryl siloxanes; polyether siloxane copolymers; amino substituted silicones such as amodimethicone with tradename BY16-872 available from Dow Corning; silicone microemulsion with tradename DC2-8194 available from Dow Corning; quaternized silicones such as that available from Union Carbide under the tradename UCAR SILICONE ALE 56 and that available from Noveon with a tradename Ultrasil Q-Plus; and mixtures thereof.

### Additional thickening polymer

The compositions of the present invention can contain additional thickening polymers. The additional thickening polymers useful herein arc those which can provide appropriate viscosity and rheology properties to the composition, so that the composition of the present composition has a suitable viscosity.

The additional thickening polymer can be included in the composition of the present invention at a level by weight of from 0.01% to 5%, still more preferably from 0.05% to 3%, even more preferably from 0.1% to 2.0%.

A variety of additional thickening polymers other than those described above under the title "HYDROPHOBICALLY MODIFIED CATIONIC THICKENING POLYMER" can be used in the compositions of the present invention. Additional thickening polymers useful herein include, for example, cellulose and its derivatives such as hydroxymethyl cellulose, hydroxyethyl cellulose, hydroxyethylethylcellulose, cetyl hydroxyethylcellulose having tradenames NATROSOL PLUS 330CS and POLYSURF 67, both available from Aqualon Company, DeI, USA; guar gums such as cationic or nonionic guar gums; crosslinked polymers such as nonionic crosslinked polymers and cationic crosslinked polymers; and acrylate polymers such as sodium polyacrylate, polyethylacrylate, and polyacrylamide; and Polyquaternium-37 available from 3V Sigma with tradenames Synthalen CR, Synthalen CU, and Synthalen CN. The thickening polymers useful herein may include the polymers disclosed below under the title "Cationic conditioning polymer".

### Cationic conditioning_polymer

The conditioning compositions of the present invention may further include cationic conditioning polymers. The cationic polymers hereof will generally have a weight average molecular weight which is at least 5,000, typically at least 10,000, and is less than about 10 million, preferably, the molecular weight is from 100,000 to 2 million. The cationic polymers useful herein may include the polymers disclosed above under the title "Additional thickening polymer".

The cationic conditioning polymer can be included in the compositions at a level by weight of preferably from 0.01% to 10%, more preferably from 0.05% to 5%.

Suitable cationic conditioning polymers include, for example: copolymers or 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16), such as those commercially available from BASF Wyandotte Corp. (Parsippany, NJ, USA) under the LUVIQUAT tradename (e.g., LUVIQUAT FC 370); copolymers of 1-vinyl-2-pyrrolidone and dimethylaminoethyl methacrylate (referred to in the industry by CTFA as Polyquaternium-11) such as those commercially available from Gaf Corporation (Wayne, NJ, USA) under the GAFQUAT tradename (e.g., GAFQUAT 755N); cationic diallyl quaternary ammonium-containing polymers, including, for example, dimethyldiallylamonium chloride homopolymer and copolymers of acrylamide and dimethyldiallylammonium chloride, referred to in the industry (CTFA) as Polyquaternium 6 and Polyquaternium 7, Polyquatemium-7 including that commercially available with a tradename Merquat 550 from Ondeo Nalco; polymethacrylamidopropyl trimonium chloride such as that commercially available with a tradename Polycare 133 from Rhone-Poulene; and Polyquaternium-37 available from 3V Sigma with tradenames Synthalen CR, Synthalen CU, and Synthalen CN.

Also suitable cationic conditioning polymers herein include cationic cellulose derivatives. Cationic cellulose derivative useful herein include, for example, salts of hydroxyethyl cellulose reacted with trimethyl ammonium substituted epoxide, referred to in the industry (CTFA) as Polyquaternium 10, available from Amerchol Corp. (Edison, NJ, USA) in their Polymer JR® and LR® series, and also available from National Starch & Chemical with a tradename Celquat SC-230M; and Polyquaternium-4 with tradename Celquat H-100 available from National Starch & Chemical.

Other suitable cationic conditioning polymers include cationic guar gum derivatives, such as guar hydroxypropyltrimonium chloride commercially available from Rhodia in their Jaguar series.

### Other additional components

The compositions of the present invention may further include other additional components. Other additional components generally are used individually at levels of from 0.001 % to 10%, preferably up to 5% by weight of the composition.

A wide variety of other additional components can be formulated into the present compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, nonionic surfactants such as glyceryl stearate available from Stepan Chemicals, hydrolysed keratin, proteins, plant extracts, and nutrients; emollients such as PPG-3 myristyl ether with tradename Varonic APM available from Goldschmidt, Trimethyl pentanol hydroxyethyl ether, PPG-11 stearyl ether with tradename Varonic APS available from Goldschmidt, Stearyl heptanoate with tradename Tegosoft SH available from Goldschmidt, Lactil (mixture of Sodium lactate, Sodium PCA, Glycine, Fructose, Urea, Niacinamide, Inositol, Sodium Benzoate, and Lactic acid) available from Goldschmidt, Ethyl hexyl palmitate with tradename Saracos available from Nishin Seiyu and with tradename Tegosoft OP available from Goldschmidt; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents such as the thioglycolates; perfumes; and sequestering agents, such as disodium ethylenediamine tetra-acetate; ultraviolet and infrared screening and absorbing agents such as octyl salicylate; antidandruff agents such as zinc pyrrithione and salicylic acid; visible particles with tradenames Unisphere and Unicerin available from Induchem AG (Switzerland); and anti-foaming agent such as that with a tradename XS63-B8929 available from GE-Toshiba Silicone.

### PRODUCT FORMS

The conditioning compositions of the present invention can be in the form of rinse-off products or leave-on products, can be transparent, translucent, or opaque, and can be formulated in a wide variety of product forms, including but not limited to creams, gels, emulsions, mousses and sprays.

The conditioning compositions of the present invention can be used for conditioning hair and/or skin by applying the compositions to the hair and/or skin. The conditioning composition of the present invention is especially suitable for hair care products such as hair conditioners and skin care products such as skin conditioners.

The conditioning compositions of the present invention are especially suitable for hair conditioners for rinse-off use. Such compositions are preferably used by following steps:
(i) after shampooing hair, applying to the hair an effective amount of the conditioning composition for conditioning the hair; and
(ii) then rinsing the hair.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention. Ingredients are identified by chemical or CTFA name, or otherwise defined below.

### Compositions (wt%)

| | Ex.1 | Ex.2 | Ex.3 | Ex.4 | Ex.5 | Ex.6 | Ex.7 |
|---|---|---|---|---|---|---|---|
| Hydrophobically modified cationic cellulose polymer-1 *1 | 0.53 | 0.8 | 0.7 | 0.6 | 0.63 | - | 0.4 |
| Hydrophobically modified cationic cellulose polymer-2 *2 | - | - | - | - | - | 1.5 | - |
| Hydrophobically modified cationic cellulose polymer-6 *6 | - | - | - | - | - | - | 0.3 |
| Cetyl trimethyl ammonium chloride *10 | 0.65 | 0.5 | - | 0.8 | 0.6 | 0.7 | 0.8 |
| Stearyl trimethyl ammonium chloride *11 | - | - | 0.6 | - | - | - | - |
| Hydrophobically substituted amidomethicone copolyol *13 | 4.0 | 5.0 | 4.5 | - | 4.0 | 3.5 | 5.0 |
| Hydrophobically substituted aminomethicone copolyol *14 | - | - | - | 5.0 | 2.0 | - | - |
| Perfume | 0.3 | 0.3 | 0.3 | 0.3 | 0.3 | 0.3 | 0.3 |
| Disodium EDTA | 0.13 | 0.13 | 0.13 | 0.13 | 0.13 | 0.13 | 0.13 |
| Methyl Paraben | 0.2 | 0.2 | 0.2 | 0.2 | 0.2 | 0.2 | 0.2 |
| Methylchloroisothiazolinone/ Methylisothiazolinone *15 | 0.033 | 0.033 | 0.033 | 0.033 | 0.033 | 0.033 | 0.033 |
| Deionized Water | ----------- q.s. to 100% ---------- | | | | | | |

### Compositions (wt%)

| | Ex. 8 | Ex. 9 | Ex.10 | Ex.11 | Ex.12 | Ex.13 | Ex.14 |
|---|---|---|---|---|---|---|---|
| Hydrophobically modified cationic cellulose polymer-1 *1 | 0.5 | - | - | - | - | - | - |
| Hydrophobically modified cationic cellulose polymer-3 *3 | 0.2 | 2 | 0.7 | 0.63 | - | - | 0.3 |
| Hydrophobically modified cationic cellulose polymer-4 *4 | - | - | - | - | 1.2 | 0.4 | - |
| Hydrophobically modified cationic cellulose polymer-6 *6 | - | - | - | - | - | - | 0.2 |
| Cetyltrimethylammonium chloride *10 | 0.6 | 1.5 | - | - | 0.8 | 0.6 | 0.75 |
| Stearyl trimethyl ammonium chloride *11 | - | - | 0.55 | - | - | - | - |
| Laureth-9 *12 | - | - | - | 0.7 | - | - | - |
| Hydrophobically substituted amidomethicone copolyol *13 | 3 | 4 | 4.5 | 5 | - | 5 | - |
| Hydrophobically substituted aminomethicone copolyol *14 | - | - | - | - | 6 | - | 4 |
| Perfume | 0.3 | 0.3 | 0.3 | 0.3 | 0.3 | 0.3 | 0.3 |
| Disodium EDTA | 0.13 | 0.13 | 0.13 | 0.13 | 0.13 | 0.13 | 0.13 |
| Methyl Paraben | 0.2 | 0.2 | 0.2 | 0.2 | 0.2 | 0.2 | 0.2 |
| Methylchloroisothiazolinone/ Methylisothiazolinone *15 | 0.033 | 0.033 | 0.033 | 0.033 | 0.033 | 0.033 | 0.033 |
| Deionized Water | ----------- q.s. to 100% ---------- | | | | | | |

### Compositions (wt%)

| | Ex.15 | Ex.16 | Ex.17 | Ex.18 | Ex.19 | Ex.20 | Ex.21 |
|---|---|---|---|---|---|---|---|
| Hydrophobically modified cationic cellulose polymer-5 *5 | 0.7 | - | - | - | - | - | - |
| Hydrophobically modified cationic cellulose polymer-6 *6 | - | 0.4 | 0.3 | - | - | - | - |
| Hydrophobically modified cationic cellulose polymer-7 *7 | - | - | - | 0.65 | - | - | - |
| Hydrophobically modified cationic guar polymer-1 *8 | - | - | - | - | 0.9 | 0.5 | - |
| Hydrophobically modified cationic guar polymer-2 *9 | - | - | - | - | - | 0.5 | 1 |
| Cetyltrimethylammonium chloride *10 | - | - | 0.65 | 0.7 | 0.7 | 0.6 | 0.9 |
| Stearyl trimethyl ammonium chloride *11 | 0.6 | 0.7 | - | - | - | - | - |
| Hydrophobically substituted amidomethicone copolyol *13 | 5 | 5 | 4 | 4 | 5 | 4 | 6 |
| Perfume | 0.3 | 0.3 | 0.3 | 0.3 | 0.3 | 0.3 | 0.3 |
| Disodium EDTA | 0.13 | 0.13 | 0.13 | 0.13 | 0.13 | 0.13 | 0.13 |
| Methyl Paraben | 0.2 | 0.2 | 0.2 | 0.2 | 0.2 | 0.2 | 0.2 |
| Methylchloroisothiazolinone/ Methylisothiazolinone *15 | 0.033 | 0.033 | 0.033 | 0.033 | 0.033 | 0.033 | 0.033 |
| Deionized Water | ----------- q.s. to 100% ---------- | | | | | | |

### Compositions (wt%)

| | Ex.22 | Ex.23 | Ex.24 | Ex.25 | Ex.26 | Ex.27 | Ex.28 |
|---|---|---|---|---|---|---|---|
| Hydrophobically modified cationic cellulose polymer-1 *1 | 0.8 | 0.6 | - | - | 0.3 | 0.6 | 0.8 |
| Hydrophobically modified cationic cellulose polymer-2 *2 | - | - | 1.5 | - | - | - | - |
| Hydrophobically modified cationic cellulose polymer-3 *3 | - | - | - | 2.5 | - | - | - |
| Hydrophobically modified cationic cellulose polymer-6 *6 | - | - | - | - | 0.3 | - | - |
| Cetyltrimethylammonium chloride *10 | 0.5 | 0.4 | 0.7 | 1.5 | 0.8 | 0.7 | - |
| Stearyl trimethyl ammonium chloride *11 | - | 0.45 | - | - | - | - | 0.4 |
| Hydrophobically substituted amidomethicone copolyol *13 | 4.5 | - | 4.0 | 4.0 | 4.0 | 4.0 | 3.0 |
| Hydrophobically substituted aminomethicone copolyol *14 | - | 5.0 | 2.0 | - | - | - | - |
| Quatemized silicone nanoemulsion *16 | - | - | - | - | - | 1.5 | - |
| Silicone microemulsion *17 | - | - | - | - | - | - | 2.0 |
| 1,2-hexanediol | 1.0 | - | - | - | 0.5 | - | 1.0 |
| Butylene glycol | - | - | - | - | 0.5 | - | - |
| PEG-12 *18 | - | - | - | 1.0 | - | - | - |
| PEG-200 *19 | - | - | 1.0 | - | - | - | - |
| Perfume | 0.3 | 0.3 | 0.3 | 0.3 | 0.3 | 0.3 | 0.3 |
| Disodium EDTA | 0.13 | 0.13 | 0.13 | 0.13 | 0.13 | 0.13 | 0.13 |
| Methyl Paraben | 0.2 | 0.2 | 0.2 | 0.2 | 0.2 | 0.2 | 0.2 |
| Methylchloroisothiazolinone/ Methylisothiazolinone *15 | 0.033 | 0.033 | 0.033 | 0.033 | 0.033 | 0.033 | 0.033 |
| Deionized Water | ----------- q.s. to 100% ---------- | | | | | | |

### Definitions of Components

*1 Hydrophobically modified cationic cellulose polymer-1: comprised by a hydroxyethyl cellulose backbone, a cationic substitution group having the formula (i), and a hydrophobic substitution group having the formula (ii): 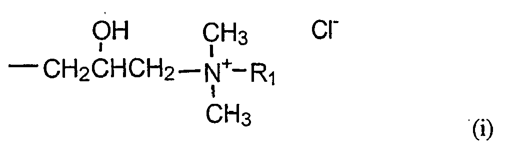 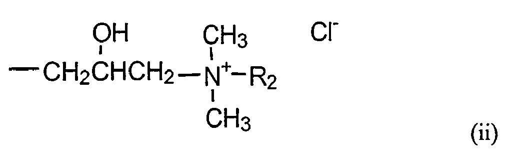 wherein R₁ is methyl and R₂ is an alkyl group having 12 carbon atoms; and having a molecular weight of about 420,000, a cationic charge density about 0.8 meq/g and a level of hydrophobic substitution about 0.115
*2 Hydrophobically modified cationic cellulose polymer-2: comprised by the same backbone and substitution groups as those for the polymer-1, and having a molecular weight of about 470,000, a cationic charge density about 1.8 meq/g and a level of hydrophobic substitution about 0.115
*3 Hydrophobically modified cationic cellulose polymer-3: comprised by the same backbone and substitution groups as those for the polymer-1, and having a molecular weight of about 1,300,000, a cationic charge density about 0.8 meq/g and a level of hydrophobic substitution about 0.115
*4 Hydrophobically modified cationic cellulose polymer-4: comprised by the same backbone and substitution groups as those for the polymer-1, and having a molecular weight of about 1,500,000, a cationic charge density about 1.8 meq/g and a level of hydrophobic substitution about 0.115
*5 Hydrophobically modified cationic cellulose polymer-5: comprised by the same backbone and substitution groups as those for the polymer-1, and having a molecular weight of about 2,200,000, a cationic charge density about 0.8 meq/g and a level of hydrophobic substitution about 0.10
*6 Hydrophobically modified cationic cellulose polymer-6: comprised by the same backbone and substitution groups as those for the polymer-1, and having a molecular weight of about 2,500,000, a cationic charge density about 1.8 meq/g and a level of hydrophobic substitution about 0.115
*7 Hydrophobically modified cationic cellulose polymer-7: comprised by the same backbone and substitution groups as those for the polymer-1, and having a molecular weight of about 2,100,000, a cationic charge density about 0.3 meq/g and a level of hydrophobic substitution-about 0.075
*8 Hydrophobically modified cationic guar polymer-1: comprised by a guar backbone and the same substitution groups as those for the cellulose polymer-1, and having a molecular weight of about 2,200,000, a cationic charge density about 1.2 meq/g and a level of hydrophobic substitution about 0.05
*9 Hydrophobically modified cationic guar polymer-2: comprised by the same backbone and substitution groups as those for the guar polymer-1, and having a molecular weight of about 400,000, a cationic charge density about 0.8 meq/g and a level of hydrophobic substitution about 0.10
*10 Cetyl trimethyl ammonium chloride: CTAC 30KC available from KCI
*11 Stearyl trimethyl ammonium chloride: Genamine STACP available from Clariant
*12 Laureth-9: BL-9EX available from Nikko Chemicals
*13 Hydrophobically modified amidomethicone copolyol: Silicone BY16-906 available from Dow Coming having the following formula: 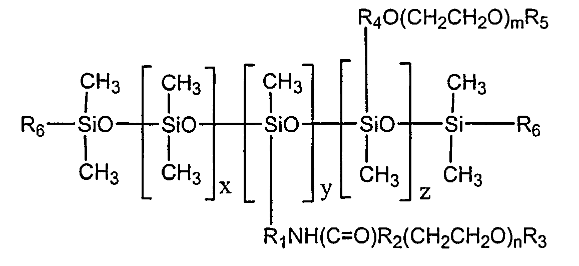 wherein R₁, R₂, and R₄ are ethyl; R₃ is an alkyl group having 12 carbon atoms; R₅ and R₆ are methyl; n is an integer of 5; m is an integer of 12; n+m = 17; x is an integer from 200 to 500; y is an integer from 5 to 40; and z is 0 or an integer from 1 to 30.
*14 Hydrophobically modified aminomethicone copolyol: that having a following formula: 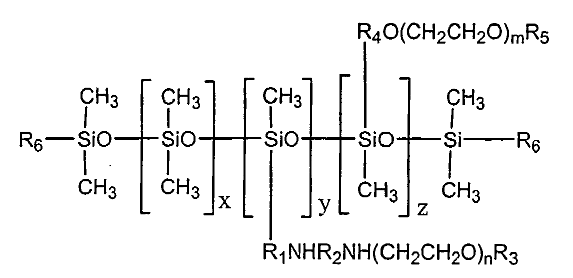 wherein R₁, R₂, R₃, R₄, R₅, R₆, n, m, n+m, x, y, and z are same as those defined above for "hydrophobically modified amidomethicone copolyol".
*15 Methylchloroisothiazolinone/ Methylisothiazolinone: Kathon CG available from Rohm&Haas
*16 Quatemized silicone nanoemulsion: DC5-7133 available from Dow Coming
*17 Silicone microemulsion: DC2-8194 available from Dow Coming
*18 PEG-12: Available from Dow Chemical
*19 PEG-200: Emkapol available from ICI

### Method of preparation

The conditioning compositions of "Ex.1" to "Ex.28" as shown above can be prepared by any conventional method well known in the art. They are suitably made as follows:

The polymeric materials are dispersed in water at room temperature, mixed with vigorous agitation, and heated to 50-75oC. Silicones, and if included, cationic surfactants and other temperature insensitive components are added to the mixture with agitation. Then the mixture is cooled down to below 40oC, and then, if included, nonionic surfactants and the remaining components such as perfumes, preservatives, and anti-foaming agents, are added to the mixture with agitation.

Examples 1 through 28 are conditioning compositions of the present invention which are particularly useful for hair conditioners for rinse-off use. These examples have many advantages.

For example, the compositions of "Ex.1" through "Ex.28" have a suitable rheology for conditioning compositions, and provide conditioning benefits, especially softness and reduced tangling on wet hair when used for hair care products such as hair conditioning products. The compositions of "Ex.1" through "Ex.28" have transparent or translucent appearance. When used for hair care products, the composition of "Ex.1" through "Ex.28" can provide the above rheological and conditioning benefits while not weighing down the hair. When used in a form of rinse-off products, the compositions of "Ex.1" through "Ex.28" can provide easy to rinse-off feel while providing the above rheological and conditioning benefits.

## Claims

1. A hair or skin conditioning composition comprising by weight:
(a) from 0.01% to 5.0%, preferably from 0.1% to 3.0%, more preferably from 0.3% to 2.0%, still more preferably from 0.4% to 0.8% of a hydrophobically modified cationic thickening polymer; wherein the hydrophobically modified cationic thickening polymer comprises a hydrophilic polymer backbone and a hydrophobic substitution group; wherein the hydrophilic polymer backbone is selected from the group consisting of a hydrophilic cellulose, a hydrophilic guar gum, a hydrophilic synthetic polymer, and mixtures thereof; and wherein the hydrophobic substitution group is cationic and has the following formula (ii): 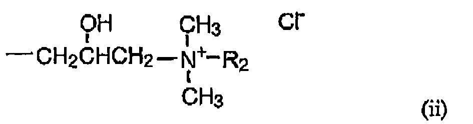 wherein R₂ is an alkyl group having from 8 to 22 carbons, and the hydrophobically modified cationic thickening polymer further comprises a cationic substitution group having the following formula (i): 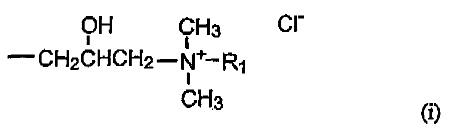 wherein R₁ is an alkyl having from 1 to 7 carbons.
(b) from 0.05% to 10.0%, preferably from 0.1% to 3.0%, more preferably from 0.2% to 2.0%, still more preferably from 0.3% to 2.0% of a surfactant selected from the group consisting of cationic surfactant, nonionic surfactant, and mixtures thereof;
(c) from 0.1% to 10%, preferably from 0.5% to 8.0%, more preferably from 0.1% to 6.0%, still more preferably from 2.0% to 5.0% of a hydrophobically modified silicone; and
(d) an aqueous carrier.

2. The conditioning composition of Claim 1 wherein the composition 1% or less of a water-insoluble high melting point oily compound.

3. The conditioning composition of Claim 1 wherein the composition is transparent or translucent, wherein the composition is preferably transparent, and wherein the composition more preferably has a transmittance of 50% or more, preferably 65% or more, more preferably 80% or more.

4. The conditioning composition of Claim 3, wherein the hydrophobically modified cationic thickening polymer comprises the cationic substitution group having the formula (i) and the hydrophobic substitution group having the formula (ii); wherein preferably R₁ is an alkyl having 1 to 3 carbon atoms, and R₂ is an alkyl group having from 10 to 18 carbon atoms; wherein more preferably R₁ is methyl and R₂ is an alkyl group having 12 carbon atoms.

5. The conditioning composition of any of the preceding claims wherein the hydrophobically modified cationic thickening polymer has a molecular weight of less than 500,000, and a level of hydrophobic substitution of from 0.10 to 0.15.

6. The conditioning composition of any of the preceding claims wherein the hydrophobically modified silicone is selected from those having an average particle size of 300nm or less in the composition, those being substantially soluble in the composition, and mixtures thereof; wherein the substantially soluble silicone compound is preferably selected from the group consisting of amino silicone copolyols, hydrophobically modified amino silicone copolyols, hydrophobically modified amido silicone copolyols, and mixtures thereof, more preferably a hydrophobically modified amido silicone copolyol, still more preferably a hydrophobically modified amido silicone copolyol having the following formula: 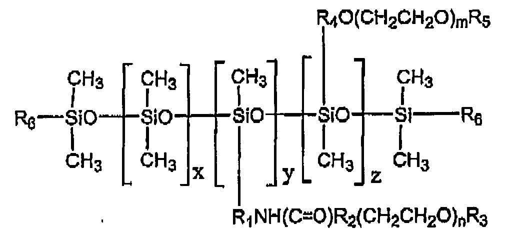 wherein R₁, R₂, R₄ are respectively C1-C3 alkyl, preferably ethyl; R₃ is an alkyl, group having from 8 to 22 carbon atoms, preferably from 10 to 20 carbon atoms, more preferably from 12 to 16 carbon atoms, still more preferably 12 carbon atoms; R₅ is H or C1-C3 alkyl, preferably methyl; R₆ is OH or CH₃, preferably methyl; n is an integer of 1-10, preferably 5; m is an integer of 2-20, preferably 12; n+m = 3-30, preferably 5-25, more preferably 8-20, still more preferably 17; x is an integer from 200 to 500, preferably from 300 to 400; y is an integer from 5 to 40, preferably from 10 to 30; and z is 0 or an integer from 1 to 30, preferably from 5 to 20.

7. The conditioning composition of any of the preceding claims further comprising a co-solvent, wherein the co-solvent is preferably selected from the group consisting of 1,2-hexane diol, hexylene glycol, butylene glycol, glycerine, and mixtures thereof.

8. The conditioning composition of any of the preceding claims which is a hair conditioning composition.

9. The conditioning composition of any of the preceding claims which is for rinse-off use.

10. A method of conditioning hair, the method comprising following steps:
(i) after shampooing hair, applying to the hair an effective amount of the conditioning composition of Claim 8; and
(ii) then rinsing the hair.

## Patentansprüche

1. Haar- oder Hautkonditionierungszusammensetzung, umfassend nach Gewicht:
(a) zu 0,01 % bis 5,0 %, vorzugsweise zu 0,1 % bis 3,0 %, mehr bevorzugt zu 0,3 % bis 2,0 %, noch mehr bevorzugt zu 0,4 % bis 0,8 % ein hydrophob modifiziertes kationisches verdickendes Polymer, wobei das hydrophob modifizierte kationische verdickende Polymer eine hydrophile Polymerhauptkette und eine hydrophobe Substitutionsgruppe umfasst; wobei die hydrophile Polymerhauptkette ausgewählt ist aus der Gruppe, bestehend aus einer hydrophilen Cellulose, einem hydrophilen Guargummi, einem hydrophilen synthetischen Polymer und Mischungen davon; und wobei die hydrophobe Substitutionsgruppe kationisch ist und die folgende Formel (ii) hat: 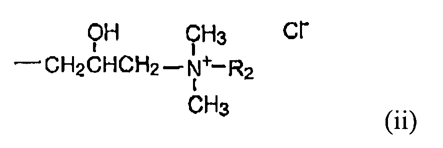 worin R₂ eine Alkylgruppe mit 8 bis 22 Kohlenstoffen ist und das hydrophob modifizierte kationische verdickende Polymer ferner eine kationische Substitutionsgruppe mit der folgenden Formel (i) umfasst: 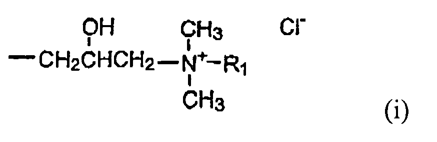 worin R₁ ein Alkyl mit 1 bis 7 Kohlenstoffen ist;
(b) zu 0,05 % bis 10,0 %, vorzugsweise zu 0,1 % bis 3,0 %, mehr bevorzugt zu 0,2 % bis 2,0 %, noch mehr bevorzugt zu 0,3 % bis 2,0 % ein Tensid, ausgewählt aus der Gruppe, bestehend aus kationischem Tensid, nichtionischem Tensid und Mischungen davon;
(c) zu 0,1 % bis 10 %, vorzugsweise zu 0,5 % bis 8,0 %, mehr bevorzugt zu 0,1 % bis 6,0 %, noch mehr bevorzugt zu 2,0 % bis 5,0 % ein hydrophob modifiziertes Silikon; und
(d) einen wässrigen Träger.

2. Konditionierungszusammensetzung nach Anspruch 1, wobei die Zusammensetzung zu 1 % oder weniger eine wasserunlösliche ölige Verbindung mit hohem Schmelzpunkt.

3. Konditionierungszusammensetzung nach Anspruch 1, wobei die Zusammensetzung transparent oder lichtdurchlässig ist, wobei die Zusammensetzung vorzugsweise transparent ist und wobei die Zusammensetzung mehr bevorzugt eine Durchlässigkeit von 50 % oder mehr, vorzugsweise 65 % oder mehr, mehr bevorzugt 80 % oder mehr aufweist.

4. Konditionierungszusammensetzung nach Anspruch 3, wobei das hydrophob modifizierte kationische verdickende Polymer die kationische Substitutionsgruppe mit der Formel (i) und die hydrophobe Substitutionsgruppe mit der Formel (ii) umfasst; worin vorzugsweise R₁ ein Alkyl mit 1 bis 3 Kohlenstoffatomen ist und R₂ eine Alkylgruppe mit 10 bis 18 Kohlenstoffatomen ist; worin mehr bevorzugt R₁ Methyl ist und R₂ eine Alkylgruppe mit 12 Kohlenstoffatomen ist.

5. Konditionierungszusammensetzung nach einem der vorstehenden Ansprüche, wobei das hydrophob modifizierte kationische verdickende Polymer ein Molekulargewicht von weniger als 500.000 und einen Grad der hydrophoben Substitution von 0,10 bis 0,15 aufweist.

6. Konditionierungszusammensetzung nach einem der vorstehenden Ansprüche, wobei das hydrophob modifizierte Silikon ausgewählt ist aus denen, die eine durchschnittliche Teilchengröße von 300 nm oder weniger in der Zusammensetzung aufweisen, denen, die in der Zusammensetzung im Wesentlichen löslich sind, und Mischungen davon; wobei die im Wesentlichen lösliche Silikonverbindung vorzugsweise ausgewählt ist aus der Gruppe, bestehend aus Aminosilikoncopolyolen, hydrophob modifizierten Aminosilikoncopolyolen, hydrophob modifizierten Amidosilikoncopolyolen und Mischungen davon, mehr bevorzugt einem hydrophob modifizierten Amidosilikoncopolyol, noch mehr bevorzugt hat ein hydrophob modifiziertes Amidosilikoncopolyol die folgende Formel: 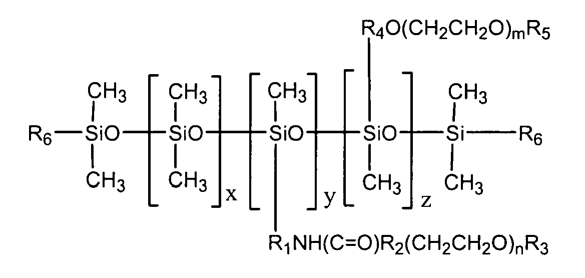 worin R₁, R₂, R₄ jeweils C1-C3-Alkyl, vorzugsweise Ethyl sind; R₃ eine Alkylgruppe mit 8 bis 22 Kohlenstoffatomen, vorzugsweise von 10 bis 20 Kohlenstoffatomen, mehr bevorzugt von 12 bis 16 Kohlenstoffatomen, noch mehr bevorzugt 12 Kohlenstoffatomen ist; R₅ H oder C1-C3-Alkyl, vorzugsweise Methyl ist; R₆ OH oder CH₃, vorzugsweise Methyl ist; n eine ganze Zahl von 1-10, vorzugsweise 5 ist; m eine ganze Zahl von 2-20, vorzugsweise 12 ist; n+m = 3-30, vorzugsweise 5-25, mehr bevorzugt 8-20, noch mehr bevorzugt 17 ist; x eine ganze Zahl von 200 bis 500, vorzugsweise von 300 bis 400 ist; y eine ganze Zahl von 5 bis 40, vorzugsweise von 10 bis 30 ist; und z 0 oder eine ganze Zahl von 1 bis 30, vorzugsweise von 5 bis 20 ist.

7. Konditionierungszusammensetzung nach einem der vorstehenden Ansprüche, die ferner ein Hilfslösungsmittel umfasst, wobei das Hilfslösungsmittel ausgewählt ist aus der Gruppe, bestehend aus 1,2-Hexandiol, Hexylenglycol, Butylenglycol, Glycerin und Mischungen davon.

8. Konditionierungszusammensetzung nach einem der vorstehenden Ansprüche, die eine Haarkonditionierungszusammensetzung ist.

9. Konditionierungszusammensetzung nach einem der vorstehenden Ansprüche, die für den Gebrauch zum Ausspülen ist.

10. Verfahren zum Konditionieren von Haar, wobei das Verfahren die folgenden Schritte umfasst:
(i) nach dem Shampoonieren des Haars Auftragen einer wirksamen Menge der Konditionierungszusammensetzung nach Anspruch 8 auf das Haar; und
(ii) anschließendes Spülen des Haars

## Revendications

1. Composition de conditionnement des cheveux ou de la peau, comprenant en poids :
(a) de 0,01 % à 5,0 %, de préférence de 0,1 % à 3,0 %, plus préférablement de 0,3 % à 2,0 %, encore plus préférablement de 0,4 % à 0,8 % d'un polymère épaississant cationique rendu hydrophobe, où le polymère épaississant cationique rendu hydrophobe comprend un squelette polymère hydrophile et un groupe de substitution hydrophobe ; où le squelette polymère hydrophile est choisi dans le groupe constitué d'une cellulose hydrophile, d'une gomme guar hydrophile, d'un polymère synthétique hydrophile, et leurs mélanges ; et où le groupe de substitution hydrophobe est cationique et présente la formule suivante (ii) : 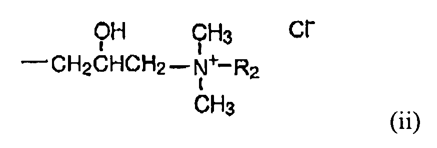 dans laquelle R₂ est un groupe alkyle ayant de 8 à 22 atomes de carbone, et le polymère épaississant cationique rendu hydrophobe comprend, en outre, un groupe de substitution cationique de formule suivante (i) : 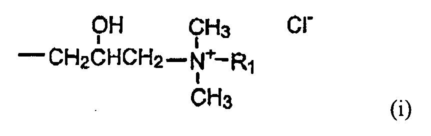 dans laquelle R₁ est un alkyle ayant de 1 à 7 atomes de carbone ;
(b) de 0,05 % à 10,0 %, de préférence de 0,1 % à 3,0 %, plus préférablement de 0,2 % à 2,0 %, encore plus préférablement de 0,3 % à 2,0 % d'un agent tensioactif choisi dans le groupe constitué des agents tensioactifs cationiques, des agents tensioactifs non ioniques, et leurs mélanges ;
(c) de 0,1 % à 10 %, de préférence de 0,5 % à 8,0 %, plus préférablement de 0,1 % à 6,0 %, encore plus préférablement de 2,0 % à 5,0 % d'une silicone rendue hydrophobe ; et
(d) un véhicule aqueux.

2. Composition de conditionnement selon la revendication 1, où la composition contient 1 % ou moins d'un composé huileux à haut point de fusion insoluble dans l'eau.

3. Composition de conditionnement selon la revendication 1, où la composition est transparente ou translucide, où la composition est de préférence transparente, et où la composition présente plus préférablement une transmittance de 50 % ou plus, de préférence de 65 % ou plus, plus préférablement de 80 % ou plus.

4. Composition de conditionnement selon la revendication 3, dans laquelle le polymère épaississant cationique rendu hydrophobe comprend le groupe de substitution cationique de formule (i) et le groupe de substitution hydrophobe de formule (ii); dans laquelle de préférence R₁ est un alkyle ayant 1 à 3 atomes de carbone et R₂ est un groupe alkyle ayant de 10 à 18 atomes de carbone ; dans laquelle plus préférablement R₁ est un méthyle et R₂ est un groupe alkyle ayant 12 atomes de carbone.

5. Composition de conditionnement de l'une quelconque des revendications précédentes, dans laquelle le polymère épaississant cationique rendu hydrophobe a une masse moléculaire inférieure à 500 000, et un niveau de substitution hydrophobe allant de 0,10 à 0,15.

6. Composition de conditionnement de l'une quelconque des revendications précédentes, dans laquelle la silicone rendue hydrophobe est choisie parmi celles ayant une taille de particule moyenne de 300 nm ou moins dans la composition, celles étant essentiellement solubles dans la composition, et les mélanges de celles-ci ; dans laquelle le composé de type silicone essentiellement soluble est de préférence choisi dans le groupe constitué des copolyols d'aminosilicone, des copolyols d'aminosilicone rendus hydrophobes, des copolyols d'amidosilicone rendus hydrophobes, et leurs mélanges, plus préférablement un copolyol d'amidosilicone rendu hydrophobe, encore plus préférablement un copolyol d'amidosilicone rendu hydrophobe de formule suivante : 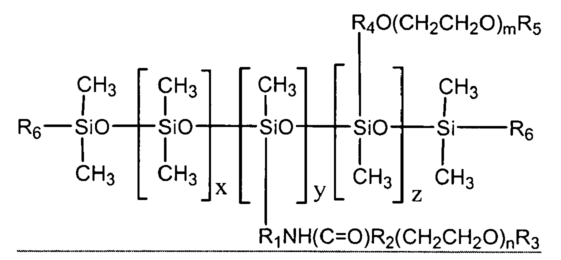 où R₁, R₂, R₄ sont respectivement un alkyle en C1 à C3 ; R₃ est un groupe alkyle ayant de 8 à 22 atomes de carbone, de préférence de 10 à 20 atomes de carbone, plus préférablement de 12 à 16 atomes de carbone, encore plus préférablement 12 atomes de carbone ; R₅ est H ou un alkyle en C1 à C3, de préférence un méthyle ; R₆ est OH ou CH₃ de préférence un méthyle ; n est un entier de 1 à 10, de préférence 5 ; m est un entier de 2 à 20, de préférence 12 ; n+m = 3 à 30, de préférence 5 à 25, plus préférablement 8 à 20, encore plus préférablement 17 ; x est un entier de 200 à 500, de préférence de 300 à 400 ; y est un entier de 5 à 40, de préférence de 10 à 30 ; et z vaut 0 ou un entier de 1 à 30, de préférence de 5 à 20.

7. Composition de conditionnement de l'une quelconque des revendications précédentes, comprenant, en outre, un cosolvant, où le cosolvant est choisi dans le groupe constitué de 1,2-hexanediol, d'hexylèneglycol, de butylèneglycol, de glycérine, et leurs mélanges.

8. Composition de conditionnement de l'une quelconque des revendications précédentes, qui est une composition de conditionnement des cheveux.

9. Composition de conditionnement de l'une quelconque des revendications précédentes, qui est destinée à une utilisation avec élimination par rinçage.

10. Procédé de conditionnement des cheveux, le procédé comprenant les étapes suivantes :
(i) après le shampoing des cheveux, on applique sur les cheveux une quantité efficace de la composition de conditionnement selon la revendication 8 ; et
(ii) puis on rince les cheveux.
